# EUROPEAN PATENT APPLICATION

(11) **EP 4 074 679 A1**
(43) Date of publication of application: **19.10.2022**
(21) Application number: 20898977.2
(22) Date of filing: 10.12.2020
(51) Int. Cl.: C04B 38/00, C12M 1/26, C12N 5/07, C12N 1/00

(54) **METHOD FOR COLLECTING LIVING TISSUE**

(30) Priority: 10.12.2019 JP 2019223086
(71) Applicant: Adamant Namiki Precision Jewel Co., Ltd., Tokyo 123-8511 (JP)
(72) Inventor: MUTO, Hikaru, Tokyo 123-8511 (JP)
(74) Representative: Dennemeyer & Associates S.A.
(86) International application number: PCT/JP2020/046096
(87) International publication number: WO 2021/117823

(57) **Abstract**

Provided is a biological tissue collection method capable of preventing damage of a biological tissue and sucking a mm-order large biological tissue. The biological tissue collection method includes the following steps. First, a component is prepared. The component is provided with multiple (n ≥ 2) holes for passing air from a first surface toward a second surface, a wall is formed between the holes, and an end portion of the wall on a first surface side is rounded and formed only of a curved surface. Next, a biological tissue is collected by the holes of the component. The biological tissue has a mm-order dimension of equal to or greater than 0.5 mm and equal to or less than 100 mm in a maximum direction, and contacts the holes on one surface by suction. Upon collection, equal to or greater than 50% and equal to or less than 90% of the area of the biological tissue is taken as as the total area of the collecting holes.

## Description

### TECHNICAL FIELD

The present invention relates to a biological tissue collection method.

### BACKGROUND ART

A skin has, an interface between a biological body and external environment, functions such as retention of liquid in the biological body, reduction in friction in water, maintenance of a body temperature at a certain temperature, and protection of the inside of the biological body from physical damage.

As such a skin, cultured skin is a biological tissue built as part of a skin structure in such a manner that epithelial cells and fibroblasts collected from a health skin piece (a piece of epithelium) is cultured in vitro. The cultured skin is distinguished as follows: skin cultured using cells collected from a health skin piece (a piece of epithelium) of a patient oneself is called autologous cultured skin (autologous epidermis), and skin cultured using cells collected from the skin (a piece of epithelium) of another person is called allogeneic cultured skin (allogeneic epidermis). The cultured skin is utilized for, e.g., treatment for serious burns, treatment for diabetic leg ulcers and venous ulcers, and treatment for cicatrices after plastic surgeries.

When a sheet-shaped biological tissue (a biological tissue including cultured epidermal cells) is used for various types of treatment as described above or culture experiment, the biological tissue needs to be picked up and collected without, e.g., any scratch, fold, or cut. However, when an attempt is made to collect the biological tissue with a tool having a sharp portion, such as tweezers, the load of the biological tissue is concentrated on the collected portion, leading to the risk of wrinkling of the biological tissue or the risk of a rupture of the biological tissue or a scratch on the biological tissue due to contact with the tool.

For this reason, the technique of collecting a biological tissue by suction without directly collecting the biological tissue with a tool itself has been devised (see, e.g., Non-Patent Literature 1 below).

Porous ceramics used for the porous vacuum chuck disclosed in Non-Patent Literature 1 is an alumina-based (Al₂O₃-based) ceramics porous body having excellent chemical stability. Further, micropores with an average pore diameter of 0.5 µm are uniformly dispersed, and therefore, when a workpiece is sucked and adhered to the porous ceramics, a suction mark formed on the workpiece due to transfer of a pore diameter shape onto the workpiece can be reduced.

### CITATION LIST

### NON-PATENT LITERATURE

Non-Patent Literature 1: "Porous Vacuum Chuck," [online], Nippon Tungsten Co., Ltd., [searched on July 4, 2019], the Internet <URL https://www.nittan.co.jp/products/ceramic_chuck_002_004.html>

### SUMMARY OF INVENTION

### PROBLEMS TO BE SOLVED BY INVENTION

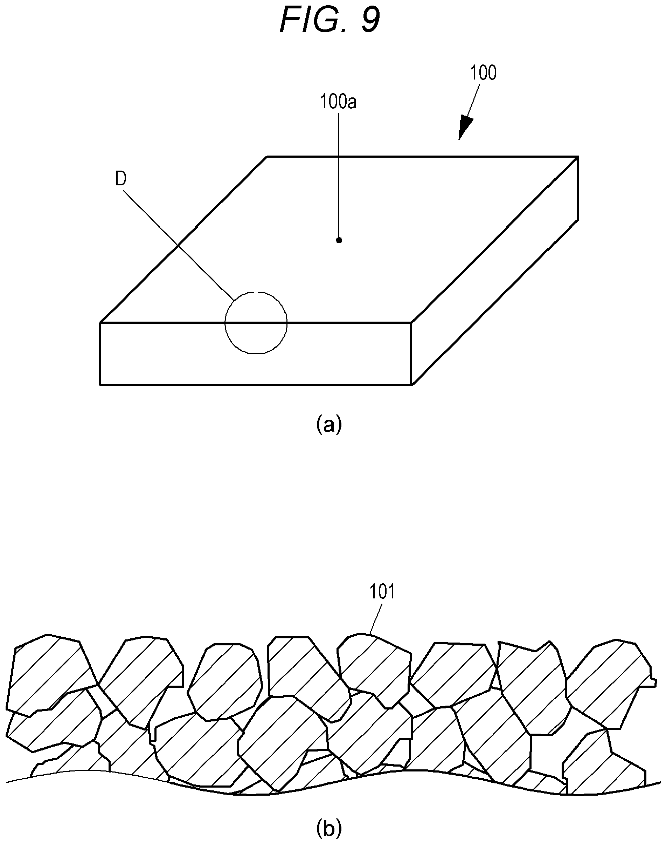

However, in porous ceramics 100 disclosed in Non-Patent Literature 1 as shown in Fig. 9(a), pointed portions of each ceramic particle 101 cannot be eliminated as shown in Fig. 9(b). Thus, there is a probability that when a biological tissue contacts a first surface 100a of the porous ceramics 100 by suction, these pointed portions damage the biological tissue.

Further, the porous ceramics 100 is not applicable to suction of a mm-order (equal to or greater than 0.5 mm) large biological tissue, and therefore, the mm-order large biological tissue cannot be sucked. Note that Fig. 9(b) shows the hatched ceramic particles 101 for the sake of easy discrimination between each ceramic particle 101 and a clearance, but is not a sectional view.

The present invention has been made in view of each of the above-described problems, and is intended to provide a biological tissue collection method capable of preventing damage of a biological tissue and sucking a mm-order large biological tissue.

### SOLUTION TO PROBLEMS

The above-described problems are solved by the following present invention. That is, a biological tissue collection method according to the present invention includes: preparing a component, the component being provided with multiple (n ≥ 2) holes for passing air from a first surface toward a second surface, a wall being formed between the holes, and an end portion of the wall on a first surface side being rounded and formed only of a curved surface; sucking a biological tissue with a dimension of equal to or greater than 0.5 mm and equal to or less than 100 mm in a maximum direction in contact with the holes on the first surface side, thereby collecting the biological tissue by the holes; and taking equal to or greater than 50% and equal to or less than 90% of an area of the biological tissue as a total area of the collecting holes.

### EFFECTS OF INVENTION

According to the biological tissue collection method according to the present invention, the component is used, in which the end portion of the wall, which is formed between the holes for sucking the biological tissue, on the first surface side is rounded and formed only of the curved surface. Thus, damage of the biological tissue when the biological tissue is collected in contact with the holes by suction can be prevented.

Further, equal to or greater than 50% and equal to or less than 90% of the area of the biological tissue sucked in contact with the holes is taken as the total area of the collecting holes, and therefore, a wide total area of the holes can be used and a sufficient suction force can be ensured. Thus, the mm-order large biological tissue can be sucked and collected.

### BRIEF DESCRIPTION OF DRAWINGS

Fig. 1 shows a schematic view of a component used for biological tissue collection in a biological tissue collection method according to the present invention.
Fig. 2(a) shows an enlarged view of a rectangular portion A of Fig. 1, and Fig. 2(b) shows a side sectional view along a chain line B-B of Fig. 2(a).
Fig. 3 shows a SEM observation image of the component used for biological tissue collection in the biological tissue collection method according to the present invention in a plane in a direction perpendicular to a hole formation direction.
Fig. 4 shows a schematic view for describing the biological tissue collection method according to the present invention.
Fig. 5(a) shows an enlarged view in a state in which a biological tissue is collected by some of the holes shown in Fig. 2(a), and Fig. 5(b) shows a side sectional view along a chain line C-C of Fig. 5(a).
Fig. 6 shows schematic views of the steps of freezing a gel body in the method for manufacturing the component used for biological tissue collection in the biological tissue collection method according to the present invention.
Fig. 7 shows a schematic sectional view of a ceramics sintered body forming the component used for biological tissue collection in the biological tissue collection method according to the present invention.
Fig. 8 shows a sectional view of the component configured such that an end portion of each wall on a first surface side as shown in Fig. 7 is rounded and formed only of a curved surface.
Fig. 9(a) shows a schematic view of porous ceramics as a component used for biological tissue collection in a typical biological tissue collection method, and Fig. 9(b) is an enlarged view of a circular portion D of Fig. 9(a).

### DESCRIPTION OF EMBODIMENTS

A first feature of the present embodiment is a biological tissue collection method including preparing a component, the component being provided with multiple (n ≥ 2) holes for passing air from a first surface toward a second surface, a wall being formed between the holes, and an end portion of the wall on a first surface side being rounded and formed only of a curved surface; sucking a biological tissue with a dimension of equal to or greater than 0.5 mm and equal to or less than 100 mm in a maximum direction in contact with the holes on the first surface side, thereby collecting the biological tissue by the holes; and taking equal to or greater than 50% and equal to or less than 90% of the area of the biological tissue as the total area of the collecting holes.

According to this biological tissue collection method, the component is used, in which the end portion of the wall, which is formed between the holes for sucking the biological tissue, on the first surface side is rounded and formed only of the curved surface. Thus, damage of the biological tissue when the biological tissue is collected in contact with the holes by suction can be prevented.

Further, equal to or greater than 50% and equal to or less than 90% of the area of the biological tissue sucked in contact with the holes is taken as the total area of the collecting holes, and therefore, a wide total area of the holes can be used and a sufficient suction force can be ensured. Thus, a mm-order large biological tissue can be sucked and collected.

A second feature of the present embodiment is the biological tissue collection method in which the component is made of ceramic.

According to this biological tissue collection method, the component is made of ceramic so that the component can have a high mechanical strength even in a case where the area of the holes of the component is set to a great value such as equal to or greater than 50% and equal to or less than 90% of the area of the biological tissue.

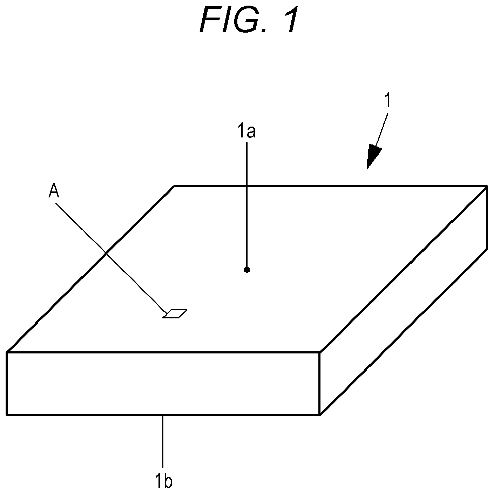

Hereinafter, an embodiment of a biological tissue collection method according to the present invention will be described with reference to Figs. 1 to 2 or Figs. 4 to 8. In the biological tissue collection method according to the present embodiment, a component 1 is used and a biological tissue 4 is collected on a first surface 1a side of the component 1 as shown in Figs. 1 to 2 and Fig. 8.

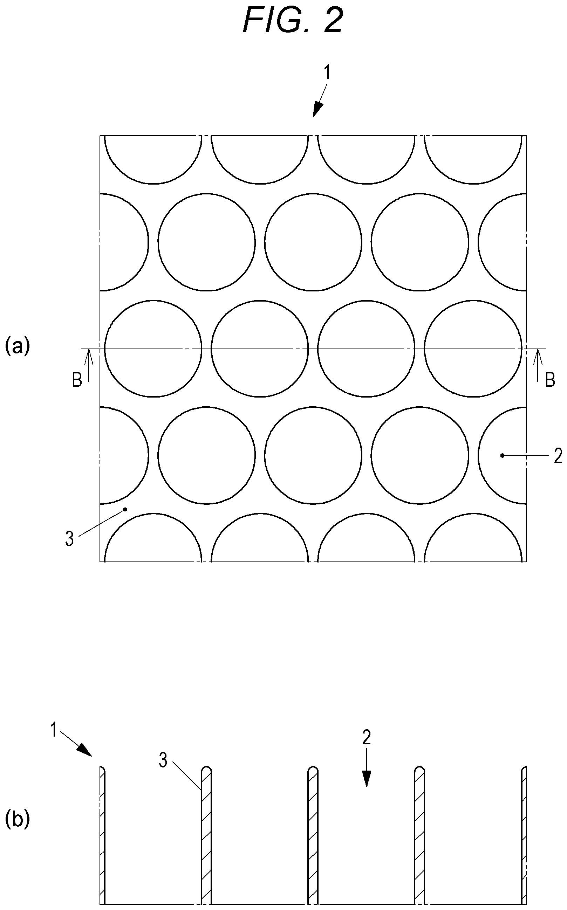

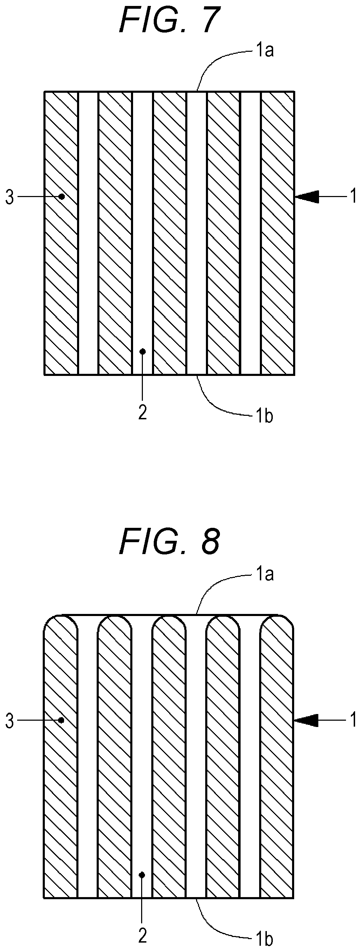

The component 1 is a sintered body (a ceramics sintered body) made of ceramic, and is provided with multiple (n ≥ 2: n is the number of holes 2) holes 2 penetrating the component 1 from a first surface 1a toward a second surface 1b. Each hole 2 appears not only on the first surface 1a but also on the second surface 1b as shown in Figs. 2 and 8. Thus, air passes between the first surface 1a and the second surface 1b through the holes 2. Round circular portions in Fig. 2(a) indicate the schematically-shown holes 2.

The diameter of the hole 2 is set to equal to or greater than 50 µm and equal to or less than 190 µm. That is, in the present embodiment, all of the diameters of the holes 2 formed in the component 1 are set within a range of equal to or greater than 50 µm and equal to or less than 190 µm. Further, the sectional shape of the hole 2 is a straight hole as shown in Fig. 8 or a not-shown tapered hole. In a case where the hole 2 is formed to have the tapered sectional shape, a diameter on the first surface 1a side as the biological tissue collection side is smaller.

The planar shape (a shape on the first surface 1a or the second surface 1b) of the hole 2 may be a circular shape schematically shown in Fig. 2(a), or may be an oval shape or a polygonal shape with m vertices (m ≥ 3). In the case of the oval shape, a long diameter is equal to or greater than 50 µm and equal to or less than 190 µm. In the case of the polygonal shape, the longest diagonal line is equal to or greater than 50 µm and equal to or less than 190 µm.

Further, a wall 3 is formed between adjacent ones of the holes 2. An end portion of the wall 3 on the first surface 1a side is rounded, and is formed only of a curved surface as shown in Fig. 2(b), 5(b), or 8. Note that the end portion does not necessarily have a certain curvature radius, and it may only be required that the end portion on the first surface 1a side is formed as a continuous curved surface with no corner or pointed portion.

Examples of the outer shape of the component 1 include a hexahedron shown in Fig. 1, but may be, e.g., a regular hexahedron, a circular columnar shape, or an oval columnar shape. Moreover, the outer dimension of the component 1 is not specifically limited. However, since the dimension of the biological tissue targeted for collection as described later in a maximum direction is assumed to be equal to or greater than 0.5 mm and equal to or less than 100 mm in the present invention, the dimension of the component 1 in a minimum direction is preferably at least 100 mm for reliably collecting the biological tissue across the entirety thereof. A thickness between the first surface 1a and the second surface 1b can be set to various values within a range not causing any interference with suction of the biological tissue, and as one example, is 1.0 mm.

The porosity of the holes 2 in the component 1 having the above-described outer shape is set to 50 ± 10%. If the porosity exceeds 50% ± 10% (i.e., 60%), it is not preferred because there is a probability that the component 1 does not have a satisfactory strength required for collecting the biological tissue. On the other hand, if the porosity is less than 50% - 10% (i.e., 40%), the number of holes 2 is insufficient due to a low porosity, and interference with suction and collection of a mm-order (equal to or greater than 0.5 mm) large biological tissue is caused.

The porosity is measurable by the Archimedes method. Moreover, the diameter of the hole 2 is measured using a scanning electron microscope (SEM) image.

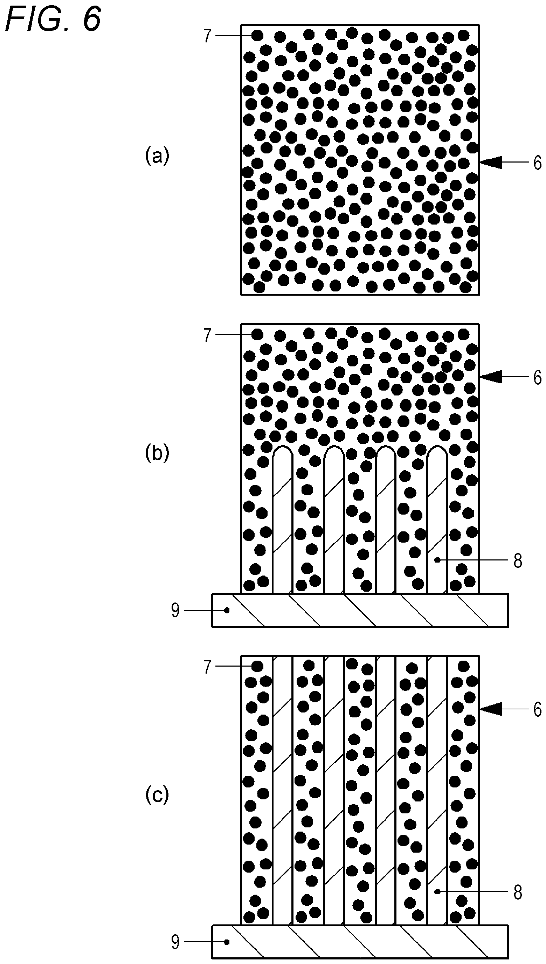

Next, the method for manufacturing the component 1 will be described as the steps of preparing the above-described component 1 with reference to Figs. 6 to 8. In the method for manufacturing the component 1, gelatable polymeric liquid is first prepared, and ceramic powder is dispersed in the liquid at a powder concentration of equal to or greater than 5% and equal to or less than 65%. In this manner, slurry is produced.

The ceramic powder dispersed in the liquid is made of zirconia (ZrO₂) and is granulated, and the average particle size of the ceramic powder is set within a range of 0.01 µm (10 nm) to 0.08 µm (80 nm). If the average particle size is less than 0.01 µm, it is not preferred because the ceramic powder is difficult to be handled and workability is degraded. On the other hand, if the average particle size exceeds 0.08 µm, it is not preferred because the ceramic powder is easily precipitated in the slurry and it is difficult to obtain stable slurry.

The water content of the slurry is 35 wt% to 95 wt%. If the water content is less than 35 wt%, the ceramic powder is easily aggregated and precipitated, and for this reason, a stable dispersion state is difficult to be held. On the other hand, if the water content exceeds 95 wt%, the density of a ceramic molded body after water has been sublimed into ice crystals is extremely low, and for this reason, it is difficult to obtain a strength for biological tissue collection.

Next, a gel body is produced in such a manner that the produced slurry is gelated. Gelation indicates that the slurry in which the ceramic powder is dispersed is solidified. Fig. 6(a) schematically shows a gel body 6. Black circles in the gel body 6 indicate dispersed ceramic powder 7.

Next, the produced gel body 6 is frozen within a range of equal to or greater than -40°C and equal to or less than -10°C. Freezing of the gel body 6 is performed in such a manner that as shown in Fig. 6(b), a bottom surface of the gel body 6 contacts a copper or aluminum freezing plate 9 and the gel body 6 is, by heat transfer, cooled in a certain direction (an upward direction in Fig. 6(b)) from the bottom surface to the other side. Note that Fig. 6(b) is a sectional view of the gel body 6 along an optional section, and the section of the gel body 6 in which the ceramic powder 7 is dispersed is shown without hatching for the sake of viewability.

Since the gel body 6 is frozen in the certain direction from the bottom surface contacting the freezing plate 9, multiple ice crystals 8 frozen in a frost column shape from water with no ceramic powder 7 being dispersed therein are formed in the certain direction in the gel body 6 as shown from Fig. 6(b) to Fig. 6(c). Note that Fig. 6(c) is also a sectional view of the gel body 6 along an optional section as in Fig. 6(b) and the section of the gel body 6 in which the ceramic powder 7 is dispersed is shown without hatching for the sake of viewability. In Figs. 6(b) and 6(c), hatched portions indicate the ice crystals 8.

Meanwhile, the ceramic powder 7 is unevenly distributed in a region of the gel body 6 other than the ice crystals 8, as shown in Figs. 6(b) and 6(c). Water expands due to freezing, and for this reason, the region in which the ceramic powder 7 is unevenly distributed is pushed and compressed by the ice crystals 8. Due to such compression, the region in which the ceramic powder 7 is unevenly distributed is densified, and accordingly, the walls 3 are formed. Note that the thickness (the dimension in the up-down direction in Fig. 6) of the gel body 6 shown in Fig. 6 is set to the thickness of the component 1.

Next, the frozen gel body 6 is dried in atmosphere, and the ice crystals 8 are sublimed. In this manner, the ceramic molded body is obtained. Thereafter, the ceramic molded body is sintered to form the component 1 including a ceramics sintered body shown in Fig. 7. Note that Fig. 7 is a sectional view of the component 1 along an optional section. A sintering method is atmospheric sintering, a heating temperature is 2°C/min to 10°C/min, a sintering temperature is 1300°C to 1500°C, the atmosphere is atmospheric air, a pressure is an ordinary pressure, and a sintering time is one hour to four hours.

As described above, the porous component 1 configured such that the multiple holes 2 are formed to extend from the first surface 1a toward the second surface 1b and the wall 3 is formed between adjacent ones of the holes 2 as shown in Fig. 7 can be formed in such a manner that the ice crystals 8 are sublimed and the resultant is sintered thereafter. It has been confirmed that all of the diameters of the holes 2 formed by the above-described manufacturing method fall within a range of equal to or greater than 50 µm and equal to or less than 190 µm.

Further, only the end portions of the walls 3 on the first surface 1a side are polished and rounded, and are formed only of the curved surfaces. In this manner, the component 1 for biological tissue collection as shown in Fig. 8 is prepared.

It has been found that as the conditions for achieving both of a porosity of 50 ± 10% and a diameter of equal to or greater than 50 µm and equal to or less than 190 µm for the hole 2, a condition that the concentration of the ceramic powder 7 dispersed in the gelatable liquid is set to equal to or greater than 5% and equal to or less than 65% and a condition that the temperature of freezing the gel body 6 is set within a range of equal to or greater than -40°C and equal to or less than -10°C are essential. If either one of the range of the concentration of the ceramic powder 7 or the range of the temperature of freezing the gel body 6 falls outside the above-described range, both of the porosity and the diameter cannot be set within the desired ranges.

If the concentration of the ceramic powder 7 is less than 5%, the density of the ceramic molded body becomes too low, and for this reason, a satisfactory strength as the component for biological tissue collection cannot be obtained. If the concentration of the ceramic powder 7 exceeds 65%, the ceramic powder is easily aggregated and precipitated, and for this reason, the stable dispersion state is difficult to be held.

If the temperature of freezing the gel body 6 is less than -40°C, the entirety of the gel body 6 is frozen before the ice crystals 8 are formed in the frost column shape, and for this reason, the ceramic powder 7 cannot be unevenly distributed in the region of the gel body 6 other than the ice crystals 8. If the temperature of freezing the gel body 6 exceeds -10°C, it is difficult to form the ice crystals 8 in the gel body 6.

Further, it is more preferred for the component 1 for biological tissue collection that the ice crystals 8 are grown in the certain direction in the gel body 6 by freezing of the gel body 6 as shown in Fig. 6 and the dense walls 3 are formed by formation of the holes 2 in the certain direction as shown in Fig. 7 or 8. The dense wall includes not only a wall with no holes, micropores, or nanopores, but also a wall with a density of equal to or greater than 99%. A reason why the dense wall is not limited to one with a density of 100% is that in addition to the frost-columnar ice crystals 8 in the gel body 6, an ice having an extremely-smaller diameter than that of the ice crystal 8 is formed in the wall in the above-described freezing step in some cases. Such an ice is also sublimed upon drying of the gel body 6. For this reason, fine holes or pores are formed after sublimation of the ice, and holes, micropores, or nanopores are formed in the wall 3 in some cases.

For formation of the holes 2 in the certain direction, the ice crystals 8 need to be formed in the certain direction in the gel body 6. As a result of study conducted by the applicant of the present application, the conditions necessary for forming the ice crystals 8 in the certain direction include a condition that the gel body 6 is, by heat transfer, gradually frozen from one location to the other location along the certain direction within a range of equal to or greater than -40°C and equal to or less than -10°C.

In Figs. 6(b) and 6(c), the surface of the gel body 6 contacting the freezing plate 9 is one location from which freezing is started, and the gel body 6 is, by heat transfer, gradually frozen toward the upper surface as the other location along the certain direction (the upward direction in Figs. 6(b) and 6(c)).

The direction of forming the holes 2 is one direction so that the ceramic powder 7 can be unevenly distributed with regularity and the ice crystals 8 can uniformly apply compression force to the entire surfaces of the walls 3. Thus, the walls 3 can be more densely formed. Thus, the component 1 can be obtained, which has the holes 2 and has high mechanical properties (mechanical strength and processability) suitable for biological tissue collection. Consequently, it is more preferred for the component for biological tissue collection that not only the porosity and diameter of the hole 2 are set, but also the direction of forming the holes 2 is set to one direction.

Further, in the present embodiment, zirconia is used as the ceramic powder 7 which is a raw material, and therefore, the ceramics sintered body is also zirconia. Since the ceramics sintered body is zirconia and has properties such as chemical durability, thermal resistance, and innoxiousness in terms of health, zirconia is suitable for the component for biological tissue collection. Further, favorable mechanical properties (mechanical strength and processability) are obtained.

For ensuring a higher mechanical strength, zirconia is more preferably yttria (yttrium oxide: Y₂O₃) containing zirconia. Specific examples include 2Y zirconia (2 mol% yttria containing zirconia), 2.5Y zirconia (2.5 mol% yttria containing zirconia), 3Y zirconia (3 mol% yttria containing zirconia), and 8Y zirconia (8 mol% yttria containing zirconia).

Note that Fig. 6 shows the holes 2 thin, considering the viewability of each particle of the ceramic powder 7. Moreover, Figs. 7 and 8 also show the holes 2 thin as in Fig. 6, considering the viewability of the walls 3.

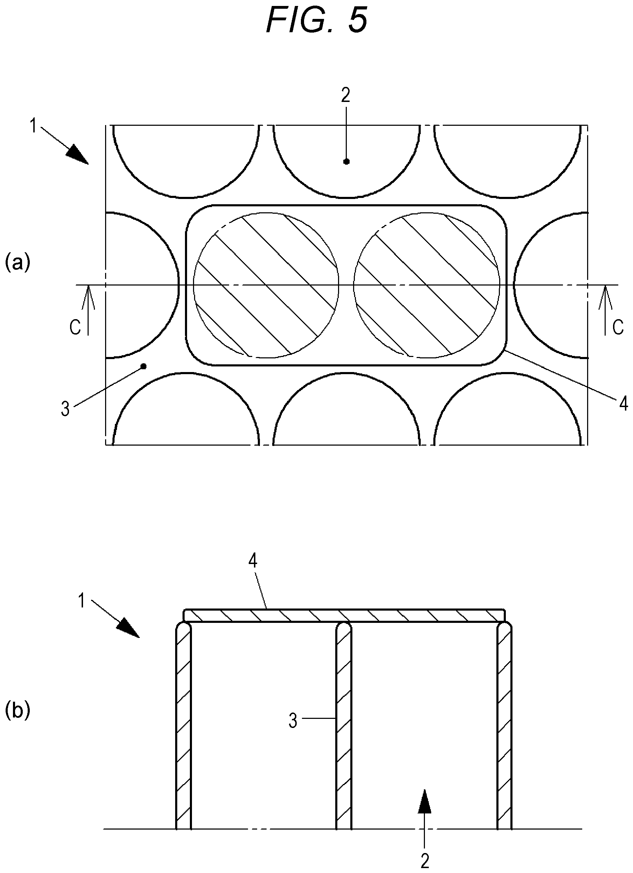

The component 1 prepared through the manufacturing method of Figs. 6 to 8 is, at side surfaces and a peripheral edge portion on the second surface 1b side, is held by a chuck 5 shown in Fig. 5. The first surface 1a of the component 1 is exposed without held or closed by the chuck 5. A portion of the second surface 1b other than the peripheral edge portion and the chuck 5 together form a clearance as an air passage therebetween.

The chuck 5 is made of, e.g., stainless steel, and is provided with a vent hole 5a. The vent hole 5a is formed in a circular shape with a diameter of about 5 mm, and is coupled to a not-shown pump such as a vacuum pump.

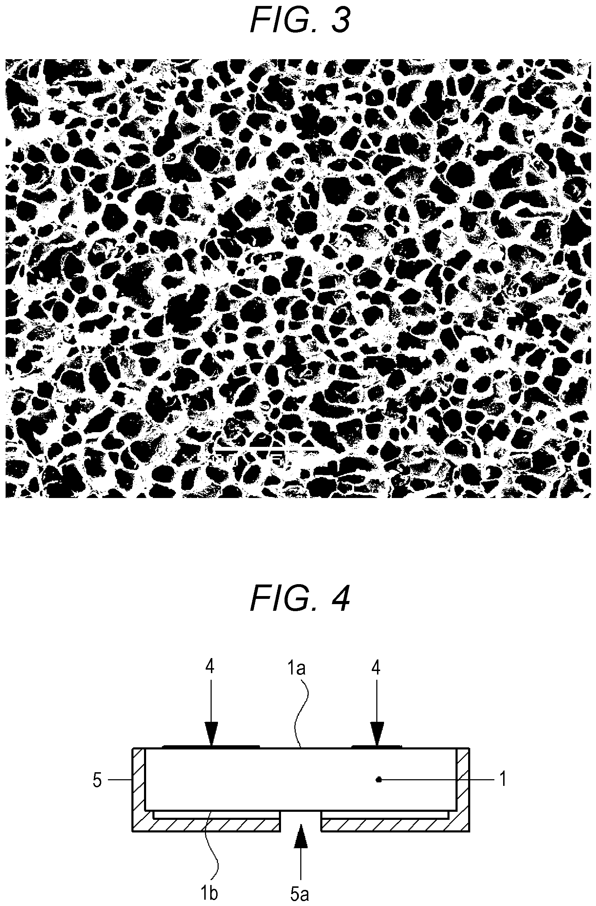

By suction (i.e., vacuuming) with the vacuum pump, suction (vacuuming) is performed from the second surface 1b toward the first surface 1a through the vent hole 5a and the clearance as the air passage. Since the first surface 1a is exposed across the entirety thereof, the biological tissue 4 is sucked onto the first surface 1a through all of the holes 2 appearing on the first surface 1a if the biological tissue 4 is present in the vicinity of the first surface 1a upon such vacuuming. Accordingly, as shown in Figs. 4 and 5, the biological tissue 4 is sucked onto the first surface 1a of the component 1 through the holes 2, and is held in contact with the holes 2 on the first surface 1a. In this manner, the biological tissue 4 is collected using the holes 2. Further, the biological tissue 4 at any location on the first surface 1a can be collected, and multiple biological tissues 4 can be collected at once as shown in Fig. 4.

The biological tissue 4 targeted for collection in the present embodiment includes cultured skin, and specifically includes mm-order cultured skin in a sheet shape. The mm-order biological tissue according to the present embodiment indicates a biological tissue with a dimension of equal to or greater than 0.5 mm and equal to or less than 100 mm in the maximum direction. The basis for such a numerical range is that 0.5 mm rounded to 1.0 mm is taken as the minimum value and a rough upper limit for culturable skin is set to 100 mm.

Such a biological tissue 4 is collected by the multiple holes 2 as shown in Fig. 5. As described above, the diameter of each hole 2 is equal to or greater than 50 µm and equal to or less than 190 µm, and the dimension of the biological tissue 4 in the maximum direction is equal to or greater than 0.5 mm and equal to or less than 100 mm. Thus, the biological tissue 4 is, with the dimension in the maximum direction, held and collected using about 2.6 to 2000 holes 2. Fig. 5 shows a state in which the biological tissue 4 is collected using two holes for the sake of viewability. Note that the dimension of the biological tissue 4 in the maximum direction in Fig. 5(a) is a dimension in a lateral direction (the horizontal direction) in Fig. 5(a).

When the biological tissue 4 is collected by the multiple holes 2, equal to or greater than 50% and equal to or less than 90% of the planar area of the biological tissue 4 is taken as the total area of the collecting holes (i.e., the holes sucking the biological tissue 4) 2. Taking Fig. 5(a) as an example, equal to or greater than 50% and equal to or less than 90% of the planar area (the area shown in Fig. 5(a)) of the substantially rectangular biological tissue 4 is taken as the total area of the holes 2 sucking the biological tissue 4 (the total area of two hatched portions).

Note that a suction pressure is set to 800 Pa or 1000 Pa as one example. Suction with a suction pressure of 800 Pa or 1000 Pa is preferred because the biological tissue 4 can be collected without damaging the biological tissue 4 or forming a suction mark on the biological tissue 4.

According to the method for collecting the biological tissue 4 in the present embodiment as described above, the component 1 is used, in which the end portion of each wall 3, which is formed between adjacent ones of the holes 2 for sucking the biological tissue 4, on the first surface 1a side is rounded and formed only of the curved surface. Thus, when the biological tissue 4 is, by suction, collected in contact with the holes 2, damage of the biological tissue 4 can be prevented.

Further, equal to or greater than 50% and equal to or less than 90% of the area of the biological tissue 4 sucked in contact with the holes 2 is taken as the total area of the collecting holes 2. Thus, a wide total area of the holes 2 can be used, and a sufficient suction force can be ensured. Thus, a mm-order (equal to or greater than 0.5 mm and equal to or less than 100 mm) large biological tissue 4 can be sucked and collected. Note that it is not preferred that the total area is less than 50% because the half or more of the total area of the biological tissue 4 is not targeted for suction and there is a probability that a sufficient suction force cannot be ensured for the mm-order biological tissue 4. If the total area exceeds 90%, the area of the sucked surface of the biological tissue 4 is too large and there is a probability that a suction mark or a wrinkle is caused or damage such as a rupture or a break is caused.

Further, one biological tissue 4 is sucked and collected by the multiple holes 2 so that drop of the biological tissue 4 can be prevented and a collection process can be stably performed. By suction using the multiple holes 2, the suction pressure generated in one hole 2 can be dispersed, and a suction mark on the biological tissue 4, a wrinkle of the biological tissue 4, and damage of the biological tissue 4 can be prevented.

Further, the component 1 is made of ceramic so that the component 1 can have a high mechanical strength even in a case where the area of the holes 2 of the component 1 is set to a great value such as equal to or greater than 50% and equal to or less than 90% of the area of the biological tissue 4.

Hereinafter, an example according to the present embodiment will be described, but the present invention is not limited only to the following example. Example

Hereinafter, the method for manufacturing a component for biological tissue collection according to the present example will be described. First, gelatable polymeric liquid was prepared, and granulated zirconia powder was dispersed in the liquid at a powder concentration of 35%. In this manner, slurry was produced. In the present example, polycrystalline zirconia as a dense body containing no yttria and having a density of 99% was used, polycrystalline zirconia being obtained by sintering for two hours within a temperature range of 1350°C to 1450°C.

Next, a gel body was produced in such a manner that the produced slurry is gelated, and a copper freezing plate 9 contacted a bottom surface of the gel body as in Fig. 6(b). Then, the gel body was cooled in a certain direction from the bottom surface to the other side by heat transfer, and was frozen at -20°C. Accordingly, multiple ice crystals 8 were formed in the certain direction as in Fig. 6(c).

Next, the frozen gel body was dried in atmosphere, and accordingly, the ice crystals 8 were sublimed. In this manner, a ceramic molded body was obtained. Further, the ceramic molded body was sintered to form a component including a ceramics sintered body. A sintering method was atmospheric sintering, a heating temperature was 2°C/min, a sintering temperature was 1350°C, the atmosphere was atmospheric air, a pressure was an ordinary pressure, and a sintering time was two hours.

A SEM observation image of the component including the obtained ceramics sintered body is shown in Fig. 3. As shown in Fig. 3, it was confirmed that holes are formed from a surface of the ceramics sintered body. Fig. 3 shows that there is a variation in the planar shape and size of the hole (in Fig. 2, such a variation is ignored). Moreover, the sectional shape of the hole was a straight hole.

As a result of measurement of the porosity of the holes of the ceramics sintered body by the Archimedes method, the porosity was 60%. Moreover, as a result of check of the diameter of the hole using a SEM observation image, it was confirmed that all of the diameters observed using SEM observation images of multiple randomly-selected locations fall within a range of equal to or greater than 50 µm and equal to or less than 190 µm.

It was also confirmed that the density of a wall is 99% and the wall is dense. Moreover, an end portion of each wall on a first surface side was rounded by polishing, and was formed only of a curved surface.

Using such a component, a cultured skin of 1 mm square was sucked and collected. A vacuum pump was used for sucking the cultured skin, and a suction pressure was 800 Pa. It was confirmed that during suction, the cultured skin does not drop and a sufficient suction force is ensured.

Further, after collection, suction was stopped, and a non-sucked surface of the cultured skin was checked. As a result, it was confirmed that the cultured skin is not damaged.

### LIST OF REFERENCE SIGNS

- 1: Component for Biological Tissue Collection
- 1a, 100a: First Surface
- 1b: Second Surface
- 2: Hole
- 3: Wall
- 4: Biological Tissue
- 5: Chuck
- 5a: Vent Hole
- 6: Gel Body
- 7: Ceramic Powder
- 8: Ice Crystal
- 9: Freezing Plate
- 10: Wall
- 100: Porous Ceramics
- 101: Ceramic Particle

## Claims

1. A biological tissue collection method comprising:
preparing a component, the component being provided with multiple (n ≥ 2) holes for passing air from a first surface toward a second surface, a wall being formed between the holes, and an end portion of the wall on a first surface side being rounded and formed only of a curved surface;
sucking a biological tissue with a dimension of equal to or greater than 0.5 mm and equal to or less than 100 mm in a maximum direction in contact with the holes on the first surface side, thereby collecting the biological tissue by the holes; and
taking equal to or greater than 50% and equal to or less than 90% of an area of the biological tissue as a total area of the collecting holes.

2. The biological tissue collection method according to claim 1, wherein
the component is made of ceramic.
